# EUROPÄISCHE PATENTSCHRIFT

(11) **EP 1 573 062 B1**
(45) Veröffentlichungstag und Bekanntmachung des Hinweises auf die Patenterteilung: **12.02.2014**
(21) Anmeldenummer: 03785579.8
(22) Anmeldetag: 15.12.2003
(51) Int. Cl.: C12Q 1/68

(54) **VERFAHREN UND VORRICHTUNG ZUR PCR-AMPLIFIKATION UND DETEKTION VON NUCLEOTIDSEQUENZEN**
METHOD AND DEVICE FOR PCR-AMPLIFICATION AND DETECTION OF NUCLEOTIDE SEQUENCES
PROCEDE ET DISPOSITIF D'AMPLIFICATION PAR PCR ET DE DETECTION DE SEQUENCES NUCLEOTIDIQUES

(30) Priorität: 19.12.2002 DE 10259819
(43) Veröffentlichungstag der Anmeldung: 14.09.2005
(73) Patentinhaber: Siemens Aktiengesellschaft, 80333 München (DE)
(72) Erfinder: GUMBRECHT, Walter, 91074 Herzogenaurach (DE); STANZEL, Manfred, 91056 Erlangen (DE)
(86) Internationale Anmeldenummer: PCT/DE2003/004136
(87) Internationale Veröffentlichungsnummer: WO 2004/057022

(56) Entgegenhaltungen:
- WO-A-00/58522
- WO-A-00/60919
- WO-A-00/62036
- WO-A-02/20833
- US-A- 5 736 257
- US-B1- 6 258 606
- FUCHS A ET AL: "A SILICON LAB-ON-CHIP FOR INTEGRATED SAMPLE PREPARATION BY PCR AND DNA ANALYSIS BY HYBRIDIZATION" ANNUAL INTERNATIONAL IEEE-EMBS SPECIAL TOPIC CONFERENCE ON MICROTECHNOLOGIES IN MEDICINE AND BIOLOGY. PROCEEDINGS, XX, XX, 2. Mai 2002 (2002-05-02), Seiten 227-231, XP001180969
- HODKO D ET AL: "DETECTION OF PATHOGENS USING ON-CHIP ELECTROCHEMICAL ANALYSIS OF PCR AMPLIFIED DNA MOLECULES" PROCEEDINGS OF THE SPIE, SPIE, BELLINGHAM, VA, US, Bd. 4265, 25. Januar 2001 (2001-01-25), Seiten 65-74, XP008000573 ISSN: 0277-786X
- SOSNOWSKI R G ET AL: "RAPID DETERMINATION OF SINGLE BASE MISMATCH MUTATIONS IN DNA HYBRIDS BY DIRECT ELECTRIC FIELD CONTROL" PROCEEDINGS OF THE NATIONAL ACADEMY OF SCIENCES OF USA, NATIONAL ACADEMY OF SCIENCE. WASHINGTON, US, Bd. 94, Februar 1997 (1997-02), Seiten 1119-1123, XP000857636 ISSN: 0027-8424
- GILLES P N ET AL: "SINGLE NUCLEOTIDE POLYMORPHIC DISCRIMINATION BY AN ELECTRONIC DOT BLOT ASSAY ON SEMICONDUCTOR MICROCHIPS" NATURE BIOTECHNOLOGY, NATURE PUBLISHING, US, Bd. 17, April 1999 (1999-04), Seiten 365-370, XP002928935 ISSN: 1087-0156

## Beschreibung

Die Erfindung betrifft ein Verfahren zur PCR-Amplifikation und Detektion von Nucleotidsequenzen. Ein solches Verfahren dient beispielsweise in der medizinischen Diagnostik dazu, infektiöse Zielsequenzen viraler oder bakterieller DNA aufzuspüren. Daneben bezieht sich die Erfindung auch auf eine zugehörige Verwendung einer geeigneten Vorrichtung zur Durchführung des Verfahrens.

Während einer PCR (Polymerase Chain Reaction) wird die zu untersuchende Probe einer zyklischen Temperaturbehandlung unterzogen, bei der DNA-Fragmente im Wesentlichen mit Hilfe eines Primerpaares und einer Polymerase vervielfältigt werden. Heute stehen für solche Analysen Verfahren zur Verfügung, bei denen die PCR auf einem Microchip durchgeführt wird, der ein Array von als "Gel-Pads" ausgebildeten Microspots aufweist (WO 01/34842 A2). Um eine fluoreszenzspektroskopische Detektion von Hybridisierungen in den Microspots zu ermöglichen, wird bei den bekannten Verfahren der Analytlösung ein markierter Primer zugesetzt.

Vom Stand der Technik sind Verfahren zur Amplifikation und Detektion von Nukleinsäuren bekannt. Dabei können Gel-Pads als hydrophile Reaktionsschichten getrennte Mikrospots auf einem Mikroarray bilden, wobei in den Gel-Pads sich Oligonukleotide befinden, die mit zu identifizierenden Zielnukleinsäuren hybridisieren können. Weiterhin ist aus der Druckschrift "Nucleic Acids Res." (1999) 27 (18) e19, Seiten 1 bis 6, bekannt, Amplifikationsreaktionen in Gel-Pads auf einem Mikroarray und Nachweisreaktionen durch Einzelbasen-Elongation durchzuführen. Im Zusammenhang damit sind weiterhin die DE 196 10 115 C2, in der ein Array mit einer Mikroelektrodenanordnung offenbart ist, und die WO 01/42508 A2, die Gel-Pads mit immobilisierten Sonden in Kontakt mit Mikroelektroden offenbart, anzuführen. Schließlich beinhaltet die WO 99/36576 A1 die Verwendung von Gel-Pads in einem Array sowie Verfahren und Systeme zu deren Herstellung, wobei sogenannte intelligente Gele als Reaktionsschichten hergestellt werden sollen.

Aus der WO 00/62036 A1 und der WO 00/60919 A2 sind Verfahren bekannt, bei welchen über einen Microchip nach einer Amplifikationsreaktion von Nukleinsäuren an elektronisch adressierbaren Fängerpositionen die Nukleinsäuren an den Fängerpositionen markiert und mit Hilfe von optischen Methoden wie z.B. Lumineszenz nachgewiesen werden.

Davon ausgehend ist es Aufgabe der Erfindung, ein verbessertes Verfahren zur Amplifikation und Detektion von Nucleotidsequenzen vorzuschlagen, mit dem ein kontinuierliches Monitoring der PCR und insbesondere eine gleichzeitige Untersuchung mehrerer Zielsequenzen oder mehrerer Mutationen einer Zielsequenz auf einfache Weise möglich ist.

Die Aufgabe wird durch die Verfahrensschritte gemäß Patentanspruch 1 gelöst. Eine zugehörige Verwendung einer geeigneten Vorrichtung ist Gegenstand des Patentanspruches 10. Weiterbildungen sind in den jeweiligen abhängigen Ansprüchen angegeben.

Beim erfindungsgemäßen Verfahren wird folgendermaßen vorgegangen:
a) es wird ein Microchip mit einem Array von mehreren Analysepositionen bildenden Microspots bereit gestellt, welche jeweils eine hydrophile Reaktionsschicht und eine darin eingebettete Mikroelektrodenanordnung umfassen, wobei die Reaktionsschicht wenigstens ein immobilisiertes, mit einer zu identifizierenden Zielsequenz eines DNA-Fragments hybridisierbares Oligonucleotid als Sondenmolekül enthält,
b) eine mehrere Zielsequenzen und PCR-Reagenzien enthaltende Analytlösung wird so auf den Microchip aufgebracht, dass sie das Array vollständig überdeckt,
c) zur Amplifikation der Zielsequenzen wird das Array einer Thermozyklisierung unterworfen,
d) Hybridisierungsereignisse an immobilisierten Sondenmolekülen einer Analyseposition werden mit Hilfe elektrischer Parameter der ihr zugeordneten Mikroelektrodenanordnung detektiert, ohne Makierung der während der PCR entstandenen Amplicons der Zielsequenz,
e) dabei wird eine Reaktionsschicht mit Kopplungsgruppen zur kovalenten Bindung von Sondenmolekülen verwendet.

Vorteilhaft bei diesem Verfahren ist zunächst, dass sich Bindungs- bzw. Hybridisierungsereignisse in einem Microspot ohne Unterbrechung der ablaufenden Reaktionszyklen und mit einem minimalen apparativen Aufwand von Beginn der PCR an detektieren lassen. Da sich in den Microspots voneinander unabhängige Elektrodenanordnungen befinden, ist jede Analyseposition einzeln adressierbar und somit mit einem bestimmten Sondenmolekül bzw. einer gesuchten Zielsequenz korrelierbar. Es kann daher eine Hybridisierung in einer Vielzahl von Microspots auf einfachste Weise gleichzeitig verfolgt werden. Bei optischer Auslesung hingegen wäre ein optisches Erfassungssystem erforderlich, das schon allein wegen der geringen Größe der Spots und deren Anordnung auf engstem Raum technisch aufwendig ist. Der technische Aufwand wird noch vergrößert, wenn Arrays mit einer großen Zahl von Microspots auszulesen sind.

Die in einem Microspot durch Hybridisierung mit immobilisierten Sondenmolekülen festgehaltenen Nucleotidsequenzen verändern aufgrund ihrer elektrischen Teilladungen elektrische Parameter wie beispielsweise den Leitwert innerhalb eines Microspots oder die Impedanz einer Elektrodenanordnung. Damit ist mit einer erfindungsgemäßen Vorrichtung aus einem Bio-Chip mit Mikroelektrodenanordnung eine elektrochemische bzw. elektrische Auswertung möglich, ohne Moleküle zur Markierung der während der PCR entstandenen Amplicons der Zielsequenz zu verwenden. Der Einbau von Markierungen in die während der PCR entstandenen Amplicons birgt vor allem bei komplexen Tests die Gefahr in sich, dass es zwischen den zur Markierung erforderlichen Stoffen und zwischen diesen und zu identifizierenden Zielsequenzen zu unerwünschten Wechselwirkungen kommt. Diese können die Untersuchungsergebnisse verfälschen.

Bei einem aus DE 196 10 115 C2 bekannten impedanzspektroskopisch auslesbaren Bio-Chip sind zwar bereits auf einem Träger mehrere interdigitale Elektrodenanordnungen vorhanden, wobei Sondenmoleküle auf den Elektroden und auf den zwischen den Elektroden angeordneten Flächen immobilisiert sind. Problematisch bei dieser Art der Detektion von Bindungsereignissen ist, dass sich die Abmessungen der Elektrodenstrukturen um Größenordnungen von molekularen Dimensionen unterscheiden. Mit noch vertretbarem technischen Aufwand lassen sich Elektroden mit einer Breite zwischen 1 und 10 µm , insbesondere von etwa 5 - 10 µm, mit einem ebensolchen Abstand und mit einer Dicke von etwa 0,1 bis 0,5 µm herstellen. Der impedanzspektroskopisch erfassbare Bereich des elektrischen Feldes einer solchen Elektrodenanordnung erstreckt sich etwa 5 bis 10 µm über die Trägeroberfläche bzw. die von der Elektrodenanordnung aufgespannte Planebene hinaus. Dagegen hat ein beispielsweise 100 Basenpaare aufweisendes Sondenmolekül eine Länge von nur etwa 30 nm, d.h. 0,3 µm. Entsprechend gering ist der Einfluss von Bindungsereignissen in einer auf der Sensorfläche bzw. den Elektroden immobilisierten monomolekularen Schicht von Sondenmolekülen auf das elektrische Feld bzw. auf die Impedanz der Elektrodenanordnung.

Dadurch, dass erfindungsgemäß die Elektrodenanordnung zumindest teilweise in eine Sondenmoleküle enthaltende und für Zielmoleküle durchlässige hydrophile Reaktionsschicht eingebettet ist, kann innerhalb der Reaktionsschicht eine viel größere Anzahl von Sondenmolekülen bzw. Zielsequenzen angesammelt werden als in einer monomolekularen Schicht. Die Folge ist eine weitaus größere Beeinflussung des elektrischen Feldes bzw. des impedanzspektroskopischen Erfassungsbereiches der Elektrodenanordnung. Ein derart ausgestalteter Bio-Chip weist eine entsprechend größere Messempfindlichkeit bzw. Sensitivität auf. Bei herkömmlichen Bio-Chips dagegen würde eine durch PCR erzielte Konzentrationserhöhung der Zielsequenzen aufgrund des geringen Angebots an Sondenmolekülen zu keiner Steigerung der Sensitivität führen.

Die bei einem erfindungsgemäßen Verfahren verwendete Reaktionsschicht muss, um eine PCR durchzuführen, bis etwa 95°C thermisch stabil sein. Thermisch stabil soll dabei bedeuten, dass die Reaktionsschicht auch bei der angegebenen Temperatur derart beschaffen ist, dass sie Sondenmoleküle festhält, dass in ihr eine Hybridisierung/Denaturierung (Aufschmelzen) von Zielsequenzen und Sondenmolekülen ungehindert stattfinden kann und dass sie auch ihre sonstigen Eigenschaften im wesentlichen beibehält. Zur Immobilisierung enthält die Reaktionsschicht vorzugsweise Polymere mit Kopplungsgruppen, an die Sondenmoleküle kovalent gebunden sind. Dadurch ist sicher gewährleistet, dass Bindungspaare aus Zielsequenzen und Sondenmolekülen bei Spülvorgängen im Anschluss an eine PCR in der Reaktionsschicht zurückgehalten werden.

Eine besonders geeignete Reaktionsschicht besteht aus einem Hydrogel. Hydrogele bilden ein wässriges Milieu in mechanisch stabiler Form, das einen Stoffaustausch mit einem überwiegend wässrigen Analyten erlaubt. Als besonders geeignet haben sich radikalisch vernetzbare Hydrogele auf Acrylamidbasis mit Maleinsäureanhydrid und/oder Glycidyl(meth)acrylat als Kopplungsgruppen erwiesen.

Bei einer weiteren bevorzugten Ausführungsform umfasst der Flachträger des Bio-Chips als Substrat Silizium(Si) und eine damit verbundene Isolierschicht, wobei letztere auf ihrer der Siliziumschicht abgewandten Seite die Elektrodenanordnung und die Reaktionsschicht trägt. Bei einer solchen Anordnung lässt sich die elektrische Verschaltung der Elektrodenanordnung unter Verwendung der von Si-Speicherchips bekannten Technologie verwirklichen.

Ein besonderer Vorteil des vorgeschlagenen Verfahrens besteht darin, dass es bei Simultan- bzw. Multiplexuntersuchungen eine größere Vielfalt an unterschiedlichen Gestaltungsmöglichkeiten erlaubt. Dies liegt u.a. daran, dass in während der PCR entstandene Amplicons keine Markierung eingebaut werden muss, was vor allem bei komplexen Tests die Gefahr in sich birgt, dass es zwischen den zur Markierung erforderlichen Stoffen und zwischen diesen und zu identifizierenden Zielsequenzen zu unerwünschten Wechselwirkungen kommt.

Weitere Einzelheiten und Vorteile der Erfindung ergeben sich aus der nachfolgenden Figurenbeschreibung von Ausführungsbeispielen anhand der Zeichnung in Verbindung mit den Patentansprüchen. Es zeigen:
- Fig. 1: eine vereinfachte perspektivische Darstellung eines einen Flachträger und ein Array von Microspots umfassenden Microchips,
- Fig. 2: einen Querschnitt durch einen Spot entsprechend Linie II-II in Fig. 1, in vergrößerter ausschnittsweiser Darstellung,
- Fig. 3: einen Ausschnitt einer einem Spot zugeordneten Elektrodenanordnung,
- Fig. 4: eine Ausführungsform eines Microchips mit einer 4-poligen Elektrodenanordnung in einer Fig. 2 entsprechenden Darstellung,
- Fig. 5: die Elektrodenanordnung des Microchips von Fig. 4 in einer Fig. 3 entsprechenden Darstellung,
- Fig. 6: eine schematische Darstellung, die eine erste Verfahrensvariante einer PCR-gestützten Analyse verdeutlicht,
- Fig. 7: eine die Wirkungsweise eines unspezifischen Primerpaares aufzeigende Schemazeichnung,
- Fig. 8: eine schematische Darstellung einer Abwandlung der ersten Verfahrensvariante,
- Fig. 9: eine schematische Darstellung einer zweiten Verfahrensvariante,
- Fig. 10: eine schematische Darstellung einer dritten Verfahrensvariante

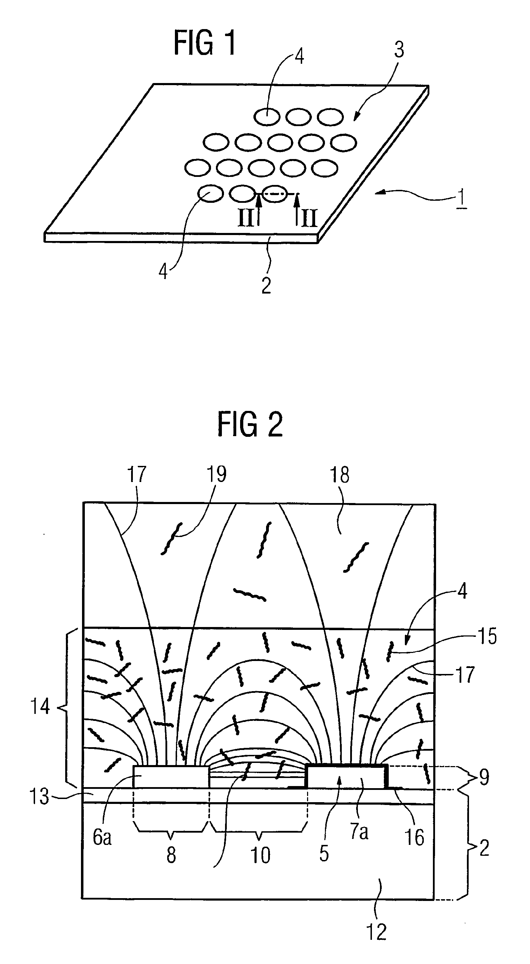

Wie Fig. 1 zeigt, umfasst ein als Bio-Chip 1 bezeichnetes Element einen Flachträger 2, auf dessen einer Seite ein Spot-Array 3 aufgebracht ist. Ein im folgenden mit Spot 4 bezeichneter Microspot enthält immobilisierte Sondenmoleküle, beispielsweise Oligonucleotide. Wird auf einen Spot 4 eine Analytlösung mit unbekannten Zielmolekülen aufgebracht, so kommt es bei entsprechender Übereinstimmung in der Basensequenz zu einer Ankopplung des Zielmoleküls an das Sondenmolekül. Die durch ein solches Bindungsereignis hervorgerufene Eigenschaftsänderung, z.B. Änderungen des spezifischen Widerstandes, der Impedanz oder der Dielektrizitätskonstante, kann mit einer Elektrodenanordnung 5 erfasst werden.

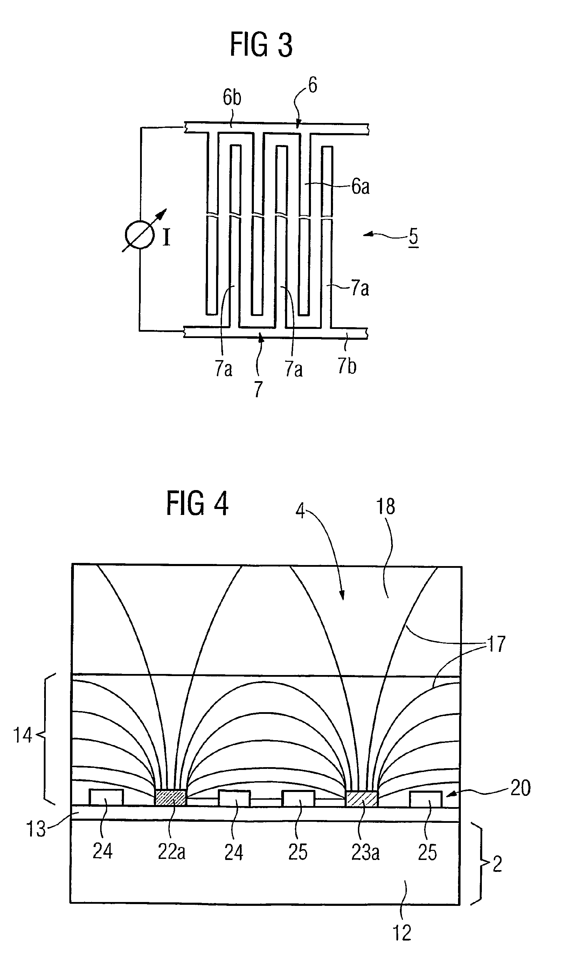

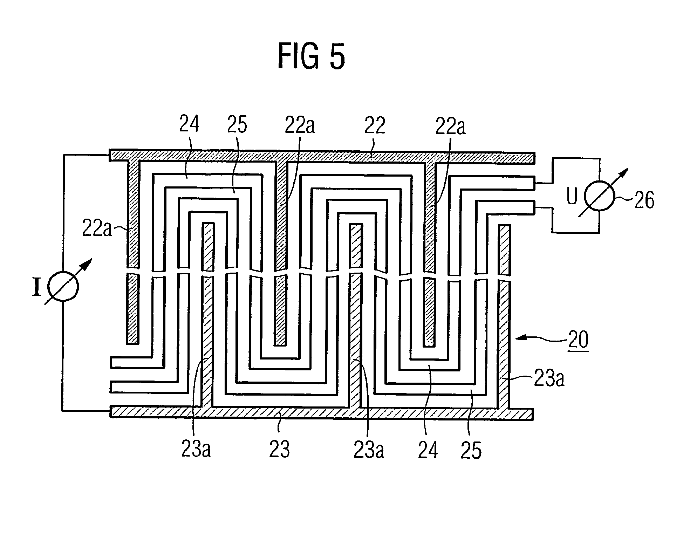

Vom Spot-Array 3 bzw. Mikrochip 1 mit darin implementierten Elektroden wird eine Vorrichtung gebildet, die ein Online-Monitoring zulässt. Eine solche Vorrichtung kann unterschiedlich ausgebildete Elektrodenanordnungen aufweisen, die in den Figuren 3 und 5 dargestellt sind. Die Figuren 2 und 4 zeigen dagegen die Phänomenologie der Immobilisierung und der Messung bei solchen Anordnungen.

Beim Ausführungsbeispiel von Fig. 2 ist eine zweipolige Elektrodenanordnung vorhanden. Diese ist beispielsweise mit Hilfe eines photolithographischen Verfahrens auf den Flachträger 2 aufgebracht. Die Elektrodenanordnung 5 umfasst zwei Elektroden 6, 7, die in Form einer Interdigitalstruktur ausgebildet sind. D.h. jede Elektrode umfasst mehrere streifenförmige parallel zueinander verlaufende Teilelektroden 6a, 7a, die sich jeweils in den Zwischenraum zweier Teilelektroden der jeweils anderen Elektrode hinein erstrecken. Die Teilelektroden 6a, 7a sind durch einen ebenfalls streifenförmigen, sich quer zu den Teilelektroden 6a, 7a streckenden Verbindungsleiter 6b, 7b miteinander verbunden. Die Elektroden 6, 7 sind mit einem hochfrequenten Wechselstrom im Megahertzbereich beaufschlagt. Die Breite 8 der Teilelektroden 6a, 7a beträgt ca. 1 µm, ihre Höhe 9 beträgt etwa 100 bis 500 nm. Zwischen den Teilelektroden 6a, 7a ist ein Abstand 10 von ebenfalls ca. 1 µm vorhanden.

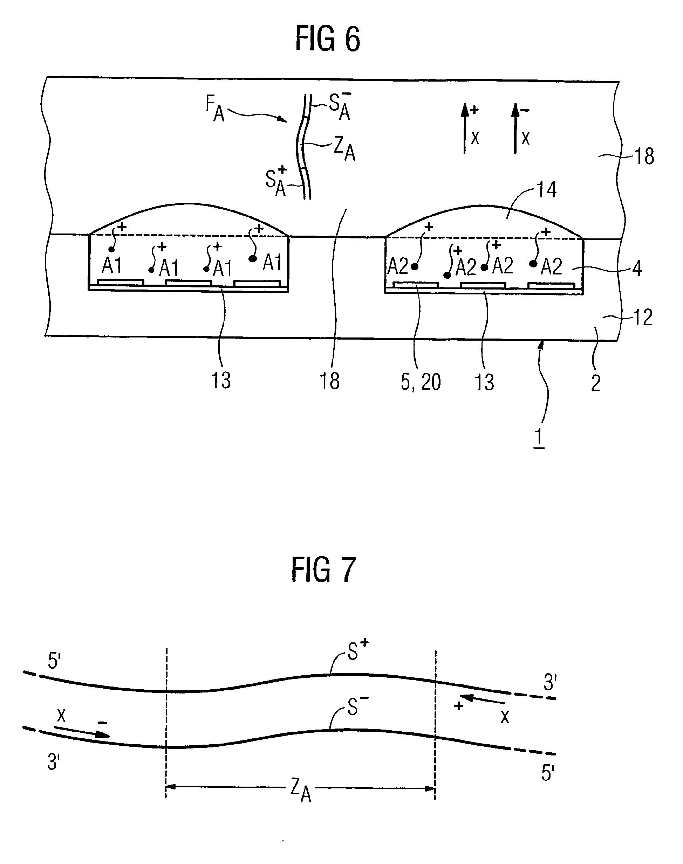

Der Flachträger 2 umfasst eine Siliziumschicht 12 und eine zwischen dieser und den Elektroden 6, 7 angeordnete Isolierschicht 13 aus einem Polymer. Die für eine z.B. impedanzspektroskopische Messung von Bindungsereignissen erforderlichen elektrischen Verschaltungen und Bauteile sind in herkömmlicher Weise durch eine entsprechende Topologie der Siliziumschicht realisiert, was in Figur 2 nicht weiter dargestellt ist. Auf der Isolierschicht 13 ist eine Reaktionsschicht 14 aus einem Hydrogel aufgebracht, welches weiter unten näher beschrieben wird. Es kann zweckmäßig sein, den Flachträger 2 bzw. die Siliziumschicht 13 im Bereich eines Spots mit einer Vertiefung zu versehen, welche mit der Reaktionsschicht 14 aufgefüllt ist (siehe Fig. 6, 8 - 10). In der Reaktionsschicht 14 bzw. dem Hydrogel sind Sondenmoleküle 15, was in Fig. 2 überdimensioniert und symbolisch dargestellt ist, eingebettet und homogen verteilt. Ein Sondenmolekül mit 300 Basen weist etwa eine Länge von 100 nm auf. Demzufolge hat eine unimolekulare Schicht von Sondenmolekülen bei herkömmlichen Microchips allenfalls etwa eine Dicke entsprechend der Linie 16 in Fig. 2. Es ist leicht einsehbar, das eine solche Schicht relativ wenige Sondenmoleküle 15 aufnehmen und dementsprechend im Falle von Bindungs- bzw. Hybridisierungsereignissen das elektrische Feld der Elektrodenanordnung nur gering beeinflussen kann. Demgegenüber ist bei einem erfindungsgemäßen Microchip der Sondenmoleküle 15 enthaltende und von Feldlinien 17 durchdrungene Reaktionsbereich wesentlich erweitert und bietet Platz für eine um mehrere Zehnerpotenzen größere Anzahl von Sondenmolekülen 15. Wird auf ein solcher Art ausgestaltetes Spot-Array 3 bzw. auf einen Spot 4 eine Analytlösung 18 aufgebracht, so finden die darin enthaltenen Zielmoleküle 19 bzw. Zielsequenzen, die in Fig. 2 ebenfalls übertrieben groß und nur symbolisch dargestellt sind, eine wesentlich größere Anzahl möglicher Bindungspartner in Form der Sondenmoleküle 15 vor.

Die Reaktionsschicht 14 ist vorzugsweise so dimensioniert bzw. weist eine derartige Dicke, beispielsweise von 5 bis 10 µm, auf, dass der impedanzspektroskopische Erfassungsbereich praktisch vollständig ausgenutzt ist, was bei einer Dicke der Reaktionsschicht von etwa 5 bis 10 µm der Fall ist. Bei entsprechender Konzentration von Sondenmolekülen 15 in diesem Bereich kann somit der bindungsspezifische Messeffekt des Microchips wesentlich erhöht werden. Die Reaktionsschicht ist so beschaffen, das sie ein wässriges Reaktionsmedium zur Verfügung stellt. Weiterhin ist sie so beschaffen, dass Zielmoleküle 19 oder auch andere für eine Reaktion benötigte Stoffe, beispielsweise Polymerase, in sie eindiffundieren können, ohne dass dabei deren Reaktivität beeinträchtigt wird.

Wie schon oben erwähnt, wird erfindungsgemäß ein Hydrogel als Reaktionsschicht 14 verwendet. Ein Hydrogel stellt ein wässriges Milieu in mechanisch stabiler Form bei gleichzeitiger Gewährleistung des Stoffaustausches in einer überwiegend wässrigen Umgebung dar. Durch Wahl der chemischen Zusammensetzung, was die Komponenten und deren Verhältnis untereinander betrifft, können die Eigenschaften der Hydrogele, wie Wassergehalt, Quellverhalten, mechanische Stabilität, etc., über weite Bereiche variiert werden. Ein Hydrogel, das leicht herstellbar ist, und das eine gute Haftung sowohl zur Elektrodenanordnung 5 als auch zur Isolierschicht 13 aufweist, ist ein radikalisch vernetzbares Hydrogel auf Acrylamidbasis, das ein Comonomer enthält, welches eine kovalente Ankopplung entsprechend modifizierter Sondenmoleküle über Linkergruppen ermöglicht. Das Hydrogel umfasst neben der Monomervorstufe des Polyacrylamids ein Vernetzungsmittel, wenigstens einen Radikalinitiator, wenigstens ein Comonomer mit reaktiven Linkergruppen und gegebenenfalls wenigstens einen Weichmacher. Nach Schichtherstellung und anschließender thermischer bzw. Fotovernetzung wird ein mit Wasser quellbares Hydrogel erhalten, das reaktive Linkergruppen zur Immobilisierung von Sondenmolekülen enthält. Als Vernetzungsmittel werden Methylenbisacrylamid und/oder Dimethylacrylsäureester, beispielsweise Tetraethylenglykoldimethacrylat, eingesetzt.

Durch die Variationen der Konzentrationen des Vernetzungsmittels lässt sich die Maschenweite des Hydrogels einstellen. Das verwendete Comonomer enthält Maleinsäureanhydrid und/oder Glycidyl(meth)acrylat. Als Weichmacher eignet sich Mono-Di- und/oder Triethylenglykol. Die genannten Ausgangsstoffe sind mit einem polaren, mit Wasser mischbaren Lösungsmittel, vorzugsweise mit Dimethylformamid, vermengt. Durch Variation des Lösungsmittelanteiles kann die Verarbeitungsviskosität eingestellt werden. Die Haftung an der Flachträgeroberfläche sowie an der Elektrodenanordnung 5 kann durch Beimengung üblicher Haftvermittler beispielsweise auf Silanbasis verstärkt werden.

In Fig. 4 und 5 ist eine vier-polare Elektrodenanordnung 20 dargestellt. Die Elektrodenanordnung 20 setzt sich aus zwei Stromelektroden 22, 23 und zwei Spannungs- bzw. Sondenelektroden 24, 25 zusammen. Die Stromelektroden 22, 23 sind entsprechend der Elektrodenanordnung 5 des Ausführungsbeispiels nach Fig. 2 angeordnet und ausgestaltet. Die Sondenelektroden 24, 25 sind ebenfalls streifenförmig und erstrecken sich als mäanderförmiger Doppelstrang durch die zwischen den Teilelektroden 22a und 23a vorhandenen Zwischenräume hindurch. Die Stromelektroden 22, 23 sind mit einem hochfrequenten Wechselstrom beaufschlagt. An den Sondenelektroden 24, 25 liegt ein Spannungsmesser 26 an, mit dem eine Veränderung des elektrischen Wechselfeldes in Folge von Hybridisierungsereignissen detektierbar ist. Die Messung kann somit unabhängig von den Stromelektroden erfolgen, so dass sich z.B. deren die Elektrodenkapazität erhöhende Polarisation nicht auf die Messung auswirken kann. Dagegen muss bei einer 2-poligen Elektrodenanordnung die Elektrodenkapazität durch eine entsprechend hohe, messtechnisch ungünstige Messfrequenz gering gehalten werden, um den für die Messung ausschlaggebenden Widerstand der Analytlösung bzw. der Reaktionsschicht bestimmen zu können.

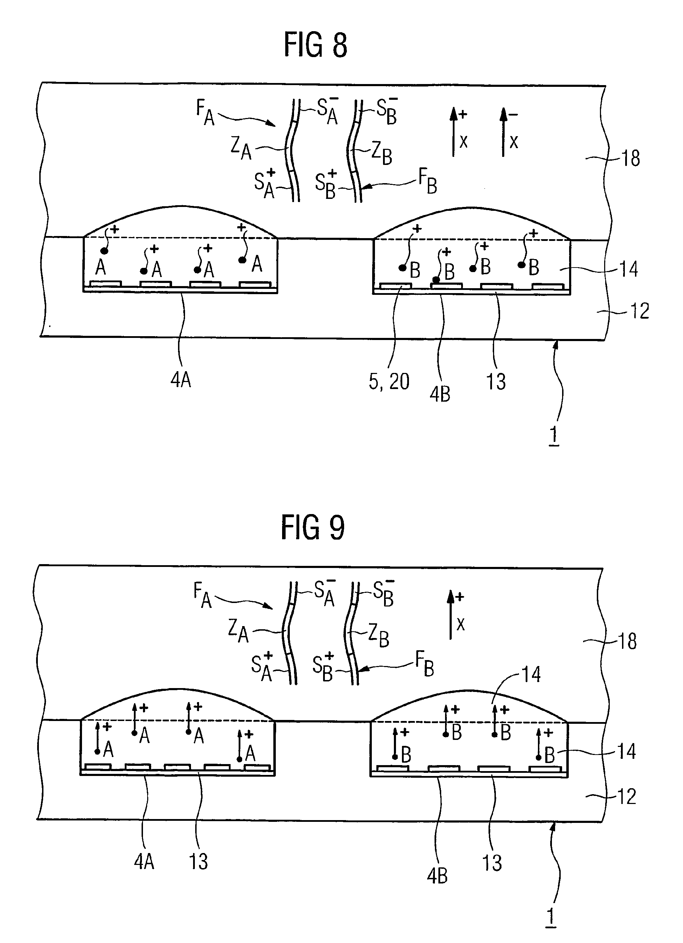

Bei einer in Fig. 6 (siehe hierzu und zu Fig. 8 bis 10 die weiter unten wiedergegebene Legende) schematisch aufgezeigten Verfahrensvariante wird auf einen Microchip 1 eine Analyselösung 18 aufgebracht, die ein DNA-Fragment FA mit einer Zielsequenz Z_{A}, ein externes Primerpaar sowie die für eine PCR erforderlichen Reagenzien, wie eine Taq-(DNA)Polymerase, dNTP's (Desoxinucleosidtriphosphate) etc. enthält. Bei der Zielsequenz Z_{A} handelt es sich um eine solche, die in mehreren unterschiedlichen Varianten vorkommen kann, z.B. Typisierung von Viren, z.B. HIV oder HPV. Jeder möglichen Variante (Z_{A1}, Z_{A2} usw.) ist mindestens ein eigener Spot 4A1, 4A2 usw. zugeordnet, wobei innerhalb der Reaktionsschicht 14 des jeweiligen Spots ein einziger Oligonucleotidtyp als Sondenmolekül immobilisiert ist, welcher mit einer spezifischen Zielsequenz hybridisieren kann. Die auf übliche Weise unter Anwendung einer Thermozyklisierung durchgeführte Amplifikation (PCR) der Zielsequenz Z_{A} erfolgt ausschließlich in der mobilen Phase 18. Vorzugsweise wird ein Primerpaar verwendet, das außerhalb der Zielsequenz Z_{A} ankoppelt (hybridisiert), wie in Fig. 7 angedeutet, ist. Der kopierte Doppelstrang, also der Strang S⁺ und der Gegenstrang S⁻ lösen sich beim Denaturieren (Aufschmelzen) voneinander. Üblicherweise wird der Strang S⁺ ("sense" Strang) zur Identifizierung einer Zielsequenz herangezogen. Dementsprechend sind in den Spots 4A1, 4A2 Oligonucleotide immobilisiert, die genau mit diesem Strang hybridisieren. Im einfachsten Fall der Anwesenheit nur eines DNA-Fragmentes Z_{A1} sammelt sich die amplifizierte Zielsequenz Z_{A1} durch Hybridisierung in jenem Spot an, in dem das entsprechend komplementäre Fängeroligonucleotid Z_{A1} immobilisiert ist.

Bei der in Fig. 8 angedeuteten Verfahrensvariante sind in der Analytlösung 18 verschiedenen Arten von DNA-Fragmenten vorhanden. Beispielhaft sind zwei solcher DNA-Fragmente, F_{A} und F_{B}, gezeigt. Bei einem oder allen der in der Analytlösung 18 vorhandenen DNA-Fragment/en kann es sich um solche gemäß der Verfahrensvariante nach Fig. 6 handeln. In diesem Fall sind unterschiedliche Gruppen von Spots vorzusehen, wobei jeder Gruppe die Typisierung der Varianten eines DNA-Fragmentes zugeordnet ist. Ziel der analytischen Untersuchung können aber auch "völlig" unterschiedliche DNA-Fragmente sein. In diesem Fall ist es prinzipiell ausreichend, einem DNA-Fragment F_{A}, F_{B} jeweils einen einzigen Analyse-Spot 4A, 4B zuzuordnen. Wie auch bei der Verfahrensvariante nach Fig. 6, ist hier in der Analytlösung 18 ein externes Primerpaar vorhanden. Dieses wird so ausgewählt, dass es für die Amplifikation aller zu analysierenden DNA-Fragmente F_{A}, F_{B} geeignet ist ("Multiplex PCR").

Die bei Fig. 6 und Fig. 8 aufgezeigten Fänger-Oligonucleotide können in weiteren Verfahrensvarianten als Primer fungieren, falls sie durch DNA-Polymerasen verlängert werden können. Es findet dann, wenn die Reaktionsschicht 18 durchlässig für die DNA-Polymerase und das/die Template/s sowie die weiteren Komponenten der PCR-Reaktion ist, eine Elongation der immobilisierten Oligonucleotide entsprechend der Sequenz der hybridisierten Matrix statt.

Bei der in Fig. 9 angedeuteten Verfahrensvariante sind mehrere verschiedene DNA-Fragmente entsprechend den Verfahrensvarianten nach Fig. 6 oder nach Fig. 8 vorhanden. Beispielhaft sind zwei solche DNA-Fragmente, F_{A} und F_{B}, gezeigt. Während bei den bisher beschriebenen Verfahrensvarianten der Analytlösung ein Primerpaar zugesetzt wurde, ist nunmehr nur ein Primer des Primerpaares mobil und gelöst in Lösung vorhanden. Dieser Primer ist unspezifisch, das heißt, es handelt sich um einen externen Primer, der bei sämtlichen in der Analytlösung vorhandenen DNA-Fragmenten F_{A} und F_{B} außerhalb der nachzuweisenden Zielsequenz Z_{A} und Z_{B} (vorzugsweise am "sense" Strang) ankoppelt. Nach dem Denaturieren der Analytlösung diffundieren die DNA-Einzelstränge wahllos in die Spots ein. In diesen Spots sind spezifische direkt vor der Zielsequenz der Analyt-DNA bindende Oligonucleotidfänger immobilisiert. Nur dort, wo die Analyt-DNA auf komplementäre immobilisierte Oligonucleotide (Fänger) trifft, findet eine Hybridisierung statt. Im folgenden Elongationsschritt der PCR wird das 5'-Ende der Fänger-Oligonucleotide, die vorher selektiv die nachzuweisenden DNA-Fragmente eingefangen (gebunden) haben, entsprechend der Information der hybridisierten Matrize verlängert. Der Fänger wird damit zum Primer für die DNA-Polymerasereaktion.

Letzteres bedeutet, dass eine Elongation nur in einem solchen Microspot stattfinden kann, in dem auch der zur Zielsequenz komplementäre Fänger vorhanden ist. Im Falle des Schemas in Fig. 9 heißt dies, dass der Strang S⁺ des DNA-Fragmentes F_{A} im Spot 4A und der Strang S⁺ des DNA-Fragmentes F_{B} im Spot 4B durch Elongation des jeweiligen immobilisierten Primers/Fängers kopiert wird. Beim nach dem jeweiligen Amplifikationsschritt und dem Aufschmelzen folgenden Reannealing lagern sich die ursprünglich vorhandenen und die in der Lösung neu entstandenen Zielsequenzen an komplementäre Fänger bzw. Sequenzen an. Auf Grund der Erhöhung der Konzentration der Zielsequenz durch den vorhergehenden PCR-Zyklus werden diese Sequenzen auch an noch nicht verlängerte, in ihrer ursprünglichen Form vorhandene Fängeroligonucleotide binden, so dass der nächste PCR-Elongationsschritt an diesen, durch die Hybridisierung neu entstandenen Primern ansetzt. Dadurch steigt die Konzentration an verlängertem Fänger mit jedem PCR-Zyklus. Diese Erhöhung der Konzentration an, um etwa 100-300 Basen verlängertem Fänger/Primer (gegenüber ursprünglich 20-30 Basen), ruft eine elektrische Feld- oder Widerstandsänderung hervor, die mit Hilfe der Elektrodenanordnung 5 bzw. 20 gemessen und zum PCR-Monitoring ("on-line" PCR) genutzt werden kann.

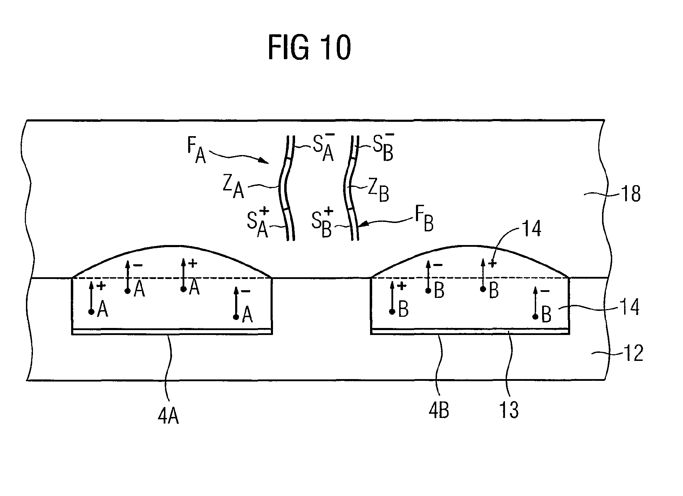

Bei der Verfahrensvariante nach Fig. 10 sind in der Analytlösung 18 eine oder mehrere DNA-Fragmentspezies entsprechend den Verfahrensvarianten nach Fig. 6 oder Fig. 8 vorhanden. Während bei den Varianten nach Fig. 6 sowie Fig. 8 Primerpaare und bei der Variante nach Fig. 9 nur ein Primer des/der Primerpaares/Primerpaare der Analytlösung zugesetzt wurden, sind hier keine gelösten freien Primer in der Lösung vorhanden. Die Elongationsreaktionen finden hier in den einzelnen Microspots 4A, 4B etc. statt. Im Gegensatz zu allen anderen Verfahrensvariante werden hier (Fig. 10) beide für den spezifischen Nachweis beider DNA-Stränge der Zielsequenz notwendigen Fänger des Fängerpaar im jeweils gleichen Gelspot immobilisiert, d.h. ein immobilisiertes internes an eine Zielsequenz hybridisierendes Fänger/Primerpaar ist vorhanden. Nach dem Aufschmelzen (Denaturieren) der Probe diffundieren die DNA-Einzelstränge wahllos in die Spots ein. Der Strang S_{A}⁺ bzw. S_{A}⁻ hybridisiert mit seinem 5'- bzw. 3'- Ende an den komplementären Primer des Primerpaares im Spot 4A.

Gemäß Fig. 10 bindet am Spot 4A der S⁺-Strang des DNA-Fragments F_{A} an einen mit A⁺ indizierten Primer sowie der S⁻-Strang des DNA-Fragments F_{A} an einen mit A⁻ indizierten Primer. Es wird dann ein antiparalleler Strang S⁻ bzw. S⁺ gebildet, der nun, da er durch Elongation des immobilisierten Primers A⁺ bzw. A⁻ synthetisiert wurde, ebenfalls immobilisiert ist. Demgegenüber ist das 3'-Ende des elongierten Stranges S⁻ bzw. S⁺ frei beweglich und kann mit einem in seiner Nähe in der Reaktionsschicht immobilisierten Gegen-Primer A⁻ bzw. A⁺ hybridisieren. Beim folgenden Amplifikationsschritt ergibt sich daraus ein S⁺- bzw. S⁻-Strang, der, da er durch Elongation des immobilisierten Primers A⁺ bzw. A⁻ gebildet wurde, ebenfalls immobilisiert ist.

Die bei jedem PCR-Zyklus auftretende Erhöhung der Konzentration an entsprechend verlängerten Fängern ruft, wie bereits weiter oben beschrieben, eine elektrische Feld- oder Widerstandsänderung hervor, die mit Hilfe der Elektrodenanordnung 5 bzw. 20 zum PCR-Monitoring genutzt werden kann. Damit ist eine on-line PCR möglich.

Für das neue, vorstehend im Einzelnen erläuterte Verfahren zur Amplifikation und Detektion von Nucleotidsequenzen wird ein Bio-Chip verwendet, wie er in den Figuren 2 und 4 in den Alternativen als Zwei-Pol- oder Vier-Pol-Anordnung beschrieben ist.

Speziell aus den Figuren 2 und 4 ist ersichtlich, dass der für das angegebene Verfahren geeignete Bio-Chip einschließlich der darauf angeordneten Reaktionsschicht und der damit in Kontakt befindlichen Analytlösung in einem in diesen Figuren als Rahmen dargestellten Gehäuse angeordnet ist. Entsprechend den Figuren 6 sowie 8 bis 10 ist das Gehäuse seitlich offen, so dass die Analytlösung im Durchfluss an der Reaktionsschicht vorbeiströmen kann.

Legende der Symbole in der vorstehenden Beschreibung und den Figuren:
- ↑: = Primer, in Lösung
- x: = unspezifisch
- A, B: = ein bestimmtes DNA-Fragment betreffend
- ⊥: = immobilisiertes Oligonucleotid, ohne Primerfunktion
- □: = immobilisierter Primer
- F: = DNA-Fragment
- S: = Einzelstrang eines DNA-Fragments
- ⁺/⁻: = einen codierenden / nicht codierenden Strang betreffend

## Patentansprüche

1. Verfahren zur Amplifikation und Detektion von Nucleotidsequenzen, mit folgenden Schritten:
a) es wird ein Microchip mit einem Array von mehreren Analysepositionen bildenden Microspots bereit gestellt, welche jeweils eine hydrophile Reaktionsschicht und eine darin eingebettete Mikroelektrodenanordnung umfasst, wobei diese wenigstens ein immobilisiertes, mit einer zu identifizierenden Zielsequenz eines DNA-Fragments hybridisierbares Oligonucleotid als Sondenmolekül enthält,
b) eine mehrere zielsequenzen und PCR-Reagenzien enthaltende Analytlösung wird so auf den Microchip aufgebracht, dass sie das Array vollständig überdeckt,
c) zur Amplifikation einer zielsequenz wird das Array einer Thermozyklisierung unterworfen,
d) Hybridisierungsereignisse an immobilisierten Sondenmolekülen einer Analyseposition werden mit Hilfe elektrischer Parameter der ihr zugeordneten Mikroelektrodenanordnung detektiert, ohne Markierung der während der PCR entstandenen Amplicons der Zielsequenz,
e) dabei wird eine Reaktionsschicht mit Kopplungsgruppen zur kovalenten Bindung von Sondenmolekülen verwendet.

2. Verfahren nach Anspruch 1, **dadurch gekennzeichnet, dass** eine hydrophile Reaktionsschicht (14) mit Kopplungsgruppen zur kovalenten Bindung von Sondenmolekülen verwendet wird.

3. Verfahren nach Anspruch 2, **dadurch gekennzeichnet, dass** als Reaktionsschicht (14) ein Hydrogel verwendet wird.

4. Verfahren nach Anspruch 2 oder 3, **dadurch gekennzeichnet, dass** ein radikalisch vernetzbares Hydrogel auf Acrylamidbasis mit Maleinsäureanhydrid und/oder Glycidyl(meth)acrylat als Kopplungsgruppen verwendet wird.

5. Verfahren nach einem der Ansprüche 1 bis 4, **dadurch gekennzeichnet, dass** ein Bio-Chip, der eine Halbleiterschicht und eine damit verbundene Isolierschicht (13) umfasst, verwendet wird, wobei letztere auf ihrer der Halbleiterschicht abgewandten Seite die Elektrodenanordnung (5) und die Reaktionsschicht (14) trägt.

6. Verfahren nach einem der Ansprüche 1 bis 5, **dadurch gekennzeichnet, dass** eine Analytlösung verwendet wird, die ein externes, außerhalb einer Zielsequenz mit einem Ziel-DNA-Strang hybridisierendes Primerpaar enthält

7. Verfahren nach einem der Ansprüche 1 bis 5, **dadurch gekennzeichnet, dass** eine Analytlösung verwendet wird, die mehrere DNA-Fragmente mit jeweils unterschiedlichen Zielsequenzen und ein einziges zur Amplifikation sämtlicher Zielsequenzen geeignetes externes Primerpaar enthält.

8. Verfahren nach einem der Ansprüche 1 bis 5, **dadurch gekennzeichnet, dass** eine Analytlösung verwendet wird, die einen mit dem einen Strang wenigstens eines DNA-Fragments zusammenwirkenden externen Primer enthält, und dass die Elongation eines Gegenstrangs innerhalb einer Reaktionsschicht mit Hilfe eines dort immobilisierten internen, mit der Zielsequenz spezifisch hybridisierenden Primers erfolgt.

9. Verfahren nach einem der Ansprüche 1 bis 5, **dadurch gekennzeichnet, dass** eine Analytlösung verwendet wird, in der mit einer Zielsequenz spezifisch hybridisierendes internes Primerpaar in einem Microspot immobilisiert ist.

10. Verwendung eines Bio-Chips mit einem Array von Analysepositionen bildenden Microspots (4), die von einer hydrophilen Reaktionsschicht (14) abgedeckt sind, zur Durchführung des Verfahrens nach einem der Ansprüche 1 bis 9.

11. Verwendung nach Anspruch 10, **dadurch gekennzeichnet, dass** der Bio-Chip mit hydrophiler Reaktionsschicht (14) in einem Gehäuse mit Durchfluss für eine Analytlösung (18) angeordnet ist.

12. Verwendung nach Anspruch 10, **dadurch gekennzeichnet, dass** der Bio-Chip als Substrat (2) Träger für die Mikrospots (4) enthält.

13. Verwendung nach Anspruch 10, **dadurch gekennzeichnet, dass** das Substrat (2) aus einem Halbleitermaterial, insbesondere Silizium (Si), auf dem eine Isolierschicht (13) aufgebracht ist, besteht.

14. Verwendung nach Anspruch 10, **dadurch gekennzeichnet, dass** der Bio-Chip ein vorgefertigter Silizium-Chip mit darin implementierten Dünnschicht-Mikroelektroden (6a, 7a; 24, 25) ist.

## Claims

1. Method for amplification and detection of nucleotide sequences, comprising the following steps:
a) providing a microchip having an array of a plurality of microspots forming analytical positions, each of which comprises a hydrophilic reaction layer and a microelectrode arrangement embedded therein, said reaction layer comprising as probe molecule at least one immobilized oligonucleotide which is hybridizable with a target sequence to be identified of a DNA fragment,
b) applying an analyte solution comprising a plurality of target sequences and PCR reagents to the microchip in such a way that it completely covers the array,
c) subjecting the array to a thermocycling process in order to amplify a target sequence,
d) detecting hybridization events on probe molecules immobilized at one analytical position with the aid of electrical parameters of the microelectrode arrangement assigned thereto, without labeling of the target sequence amplicons produced during the PCR,
e) a reaction layer having coupling groups for covalent binding of probe molecules using in the process.

2. Method according to Claim 1, **characterized in that** a hydrophilic reaction layer (14) having coupling groups for covalent binding of probe molecules is used.

3. Method according to Claim 2, **characterized in that** the reaction layer (14) used is a hydrogel.

4. Method according to Claim 2 or 3, **characterized in that** a free-radically crosslinkable hydrogel based on acrylamide with maleic anhydride and/or glycidyl (meth)acrylate as coupling groups is used.

5. Method according to any of Claims 1 to 4, **characterized in that** a biochip comprising a semiconductor layer and an insulating layer (13) connected therewith is used, the side of the latter, which faces away from the semiconductor layer, carrying the electrode arrangement (5) and the reaction layer (14).

6. Method according to any of Claims 1 to 5, **characterized in that** an analyte solution is used which comprises an external primer pair, which hybridizes with a target DNA strand outside a target sequence.

7. Method according to any of Claims 1 to 5, **characterized in that** an analyte solution is used which comprises a plurality of DNA fragments, each of which has a different target sequence, and a single external primer pair suitable for the amplification of all target sequences.

8. Method according to any of Claims 1 to 5, **characterized in that** an analyte solution is used which comprises an external primer acting together with the one strand of at least one DNA fragment and **in that** a counter strand is elongated within a reaction layer with the aid of an internal primer, which specifically hybridizes with the target sequence, immobilized there.

9. Method according to any of Claims 1 to 5, **characterized in that** an analyte solution is used in which an internal primer pair specifically hybridizing with a target sequence is immobilized in a microspot.

10. Use of a biochip having an array of microspots (4) which form analytical positions and which are covered by a hydrophilic reaction layer (14), for carrying out the method according to or in any of Claims 1 to 9.

11. Use according to Claim 10, **characterized in that** the biochip with hydrophilic reaction layer (14) is arranged in a housing having an opening for an analyte solution (18).

12. Use according to Claim 10, **characterized in that** the biochip contains carriers for the microspots (4) as substrate (2).

13. Use according to Claim 10, **characterized in that** the substrate (2) consists of a semiconductor material, in particular silicon (Si), to which an insulating layer (13) has been applied.

14. Use according to Claim 10, **characterized in that** the biochip is a prefabricated silicon chip having thin-layer microelectrodes (6a, 7a; 24, 25) implemented therein.

## Revendications

1. Procédé d'amplification et de détection de séquences de nucléotides comprenant les stades suivantes :
a) on se procure une micropuce ayant une matrice de plusieurs microtaches formant des positions d'analyse, qui comprend respectivement une couche de réaction hydrophile et un agencement de microélectrode qui y est incorporé, cette couche contenant comme molécule sonde au moins un oligonucléotide immobilisé hybridable avec une séquence cible à identifier d'un fragment d'ADN,
b) on dépose sur la micropuce une solution d'analyte contenant plusieurs cibles et réactifs PCR de manière à ce qu'elle recouvre complètement la matrice,
c) on soumet pour l'amplification d'une séquence cible la matrice a une thermocyclisation,
d) on détecte des hybridations sur les molécules sondes immobilisées d'une position d'analyse à l'aide d'un paramètre électrique de l'agencement de microélectrode qui lui est associé sans marquage de l'amplicon de la séquence cible créé pendant la PCR,
e) on utilise une couche de réaction ayant des groupes de couplage pour la liaison par covalence de molécules sondes.

2. Procédé suivant la revendication 1, **caractérisé en ce qu'**on utilise une couche ( 14 ) de réaction hydrophile ayant des groupes de couplage pour la liaison par covalence de molécules sondes.

3. Procédé suivant la revendication 2, **caractérisé en ce qu'**on utilise un hydrogel comme couche ( 14 ) de réaction.

4. Procédé suivant la revendication 2 ou 3, **caractérisé en ce qu'**on utilise un hydrogel réticulable par des radicaux à base d'acrylamide avec de l'anhydrique maléique et/ou du ( méth ) acrylate de glycidyle comme groupes de couplage.

5. Procédé suivant l'une des revendications 1 à 4, **caractérisé en ce qu'**on utilise une biopuce qui comprend une couche semiconductrice et une couche ( 13 ) isolante qui y est reliée, cette dernière portant sur sa face éloignée de la couche semiconductrice l'agencement ( 5 ) d'électrode et la couche ( 14 ) de réaction.

6. Procédé suivant l'une des revendications 1 à 5, **caractérisé en ce qu'**on utilise une solution d'analyte qui contient une paire d'amorces externes, s'hybridant en dehors d'une séquence cible avec un brin d'ADN cible.

7. Procédé suivant l'une des revendications 1 à 5, **caractérisé en ce qu'**on utilise une solution d'analyte, qui contient plusieurs fragments d'ADN ayant des séquences cibles respectivement différentes et une paire d'amorces extérieure unique, appropriée à l'amplification de toutes les séquences cibles.

8. Procédé suivant l'une des revendications 1 à 5, **caractérisé en ce qu'**on utilise une solution d'analyte, qui contient une amorce externe coopérant avec le brin d'au moins un fragment d'ADN et **en ce que** l'allongement d'un brin antagoniste s'effectue à l'intérieur d'une couche de réaction à l'aide d'une amorce interne qui y est immobilisée et qui s'hybride spécifiquement à la séquence cible.

9. Procédé suivant l'une des revendications 1 à 5, **caractérisé en ce qu'**on utilise une solution d'analyte, dans laquelle une paire d'amorces interne, s'hybridant spécifiquement à une séquence cible est immobilisée dans une microtache.

10. Utilisation d'une biopuce ayant une matrice de microtaches ( 4 ) formant des positions d'analyse, qui sont recouvertes d'une couche ( 14 ) de réaction hydrophile pour effectuer le procédé selon l'une des revendications 1 à 9.

11. Utilisation suivant la revendication 10, **caractérisée en ce que** la biopuce ayant une couche ( 14 ) de réaction hydrophile est disposée dans une boîte ayant un passage pour une solution ( 18 ) d'analyte.

12. Utilisation suivant la revendication 10, **caractérisée en ce que** la biopuce contient comme substrat ( 2 ) un support des microtaches ( 4 ).

13. Utilisation suivant la revendication 10, **caractérisée en ce que** le substrat est en un matériau semiconducteur notamment en silicium ( Si ) sur lequel est disposée une couche ( 13 ) isolante.

14. Utilisation suivant la revendication 10, **caractérisée en ce que** la biopuce est une puce de silicium, fabriquée à l'avance, ayant des microélectrodes ( 6a, 7a ; 24, 25 ) en couche mince, qui y sont mises en oeuvre.
